# EUROPEAN PATENT APPLICATION

(11) **EP 4 442 688 A1**
(43) Date of publication of application: **09.10.2024**
(21) Application number: 22900320.7
(22) Date of filing: 22.11.2022
(51) Int. Cl.: C07D 491/22

(54) **METHOD FOR PREPARING DRUG-LINKER CONJUGATE**

(30) Priority: 02.12.2021 CN 202111473108
(71) Applicant: Sichuan Kelun-Biotech Biopharmaceutical Co., Ltd., Chengdu, Sichuan 611138 (CN)
(72) Inventor: HAN, Weibiao, Chengdu, Sichuan 611138 (CN); CHEN, Xing, Chengdu, Sichuan 611138 (CN); SONG, Yaoyao, Chengdu, Sichuan 611138 (CN); HUANG, Zhongchao, Chengdu, Sichuan 611138 (CN); XIA, Dongliang, Chengdu, Sichuan 611138 (CN); TAN, Shijie, Chengdu, Sichuan 611138 (CN); WANG, Tao, Chengdu, Sichuan 611138 (CN); LIU, Zhihuan, Chengdu, Sichuan 611138 (CN); QI, Hongxia, Chengdu, Sichuan 611138 (CN); LIANG, Yufeng, Chengdu, Sichuan 611138 (CN); SONG, Hongmei, Chengdu, Sichuan 611138 (CN); GE, Junyou, Chengdu, Sichuan 611138 (CN); WANG, Jingyi, Chengdu, Sichuan 611138 (CN)
(74) Representative: Jones Day
(86) International application number: PCT/CN2022/133439
(87) International publication number: WO 2023/098515

(57) **Abstract**

The present application provides a method for preparing a drug-linker conjugate. Specifically, the present application provides a method for preparing compound IM2, which comprises the following steps: adding compound SM1 to a mixed solution of triphosgene and 4-dimethylaminopyridine to obtain an intermediate solution; adding compound SM2 to the intermediate solution, stirring the mixture, thereby obtaining compound IM2. The stability, practicality and scalability of the process are improved by optimizing the preparation process of IM2 and using a normal chromatography purification method that is easily scalable.

## Description

The present application is based on and claims priority of the application with CN application number 202111473108.9 filed on December 2, 2021, the disclosure of which is hereby incorporated into the present application in its entirety.

### TECHNICAL FIELD

The present application relates to the technical field of drug synthesis, and in particular to a method for preparing a drug-linker conjugate. The drug-linker conjugate can be used to prepare an antibody-drug conjugate.

### BACKGROUND

ADC technology couples monoclonal antibodies and drug molecules together through linkers to achieve the targeted delivery of drug molecules to target tissues using the antibody's specificity to exert their effects, thereby reducing systemic toxic side effects of the drug, increasing therapeutic window of the drug and expanding the therapeutic potential of the antibody.

The preparation process of compounds such as drug-linker conjugates has an important influence on the final product, such as the stability of the final product and the subsequent process scale-up. Process scale-up is different from laboratory operation and has certain difficulties. For example, WO2019114666A1 discloses a preparation example of the compound represented by TL033, but the preparation method (step 2) disclosed in this example is more suitable for laboratory gram-scale production. The purification of all the compounds involved uses high performance liquid chromatography, which is not suitable for industrial scale-up.

### SUMMARY OF THE INVENTION

The present application provides a method for preparing a drug-linker conjugate of an antibody-drug conjugate as shown in formula I and its intermediates. The preparation method of the present application improves the stability, practicality and scalability of the process by optimizing the preparation process of IM2 and using a normal chromatography purification method that is easily scalable.

In one aspect, the present application provides a method suitable for industrial preparation of compound IM2, comprising the following steps:
adding compound SM1 to a mixed solution of triphosgene and 4-dimethylaminopyridine to obtain an intermediate solution;
adding compound SM2 to the intermediate solution and stirring the mixture to obtain compound IM2; wherein,
compounds IM2, SM1 and SM2 have the following structures, respectively:

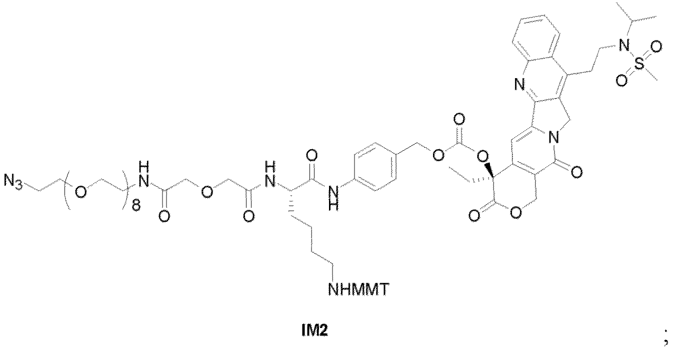

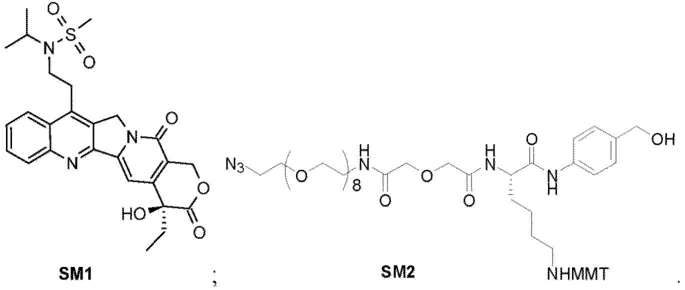

The intermediate refers to compound IM1, the structure of which is shown below:

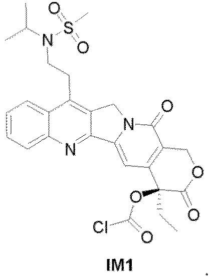

The study found that the amount of triphosgene used has a significant effect on the conversion of compound SM1 to the active intermediate IM1. In some embodiments, the feed ratio of triphosgene to compound SM1 is <1:1 in terms of molar ratio, preferably (0.3-0.9):1, more preferably (0.4-0.7):1, for example 0.4:1, 0.45:1, 0.5:1, 0.55:1, 0.6:1, 0.65:1, or 0.7:1. In some preferred embodiments, the feed ratio of triphosgene to compound SM1 is (0.4-0.7):1, preferably (0.5-0.7):1 in terms of molar ratio.

In some embodiments, the feed ratio of triphosgene to compound SM1 is <2:1 in terms of molar ratio.

The catalytic effect of 4-dimethylaminopyridine can significantly promote the transformation of compound SM1 into the active intermediate IM1. In some embodiments, the feeding ratio of 4-dimethylaminopyridine to compound SM1 is >3:1, preferably (3.5-5):1, more preferably (3.7-4.5):1, such as 3.7:1, 3.8:1, 3.9:1, 4.0:1, 4.1:1, 4.2:1, 4.3:1, 4.4:1, or 4.5:1 in terms of molar ratio. In some preferred embodiments, the feed ratio of 4-dimethylaminopyridine to compound SM1 is (3.5-4.5):1, preferably (3.7-4.5):1 in terms of molar ratio.

In some embodiments, the feed ratio of 4-dimethylaminopyridine to compound SM1 is >1:1 in terms of molar ratio.

In some embodiments, SM1 is added within the range of ±40 °C, preferably within the range of ±30 °C.

In some embodiments, SM1 is added within the range of ±25 °C, such as within the range of ±20 °C, within the range of ±15 °C, within the range of ±10 °C, or within the range of ±5 °C. In some preferred embodiments, SM1 is added within the range of ±5 °C.

In some embodiments, the feed ratio of SM1 to SM2 is 1:(0.2-4.0) , preferably 1:(0.5-2.0) in terms of molar ratio.

In some embodiments, the feed ratio of SM1 to SM2 is 1:(1-2.0), for example 1:1.1, 1:1.2, 1:1.3, 1:1.4, 1:1.5, 1:1.6, 1:1.7, 1:1.8, 1:1.9, or 1:2.0 in terms of molar ratio. In some embodiments, a dichloromethane solution of SM2 is added to the intermediate solution. Preferably, SM2 is dissolved in 5-10 times (e.g., 5 times, 6 times, 7 times, 8 times, 9 times, or 10 times) its weight of dichloromethane.

In some embodiments, stirring is carried out for 30 min-6 h after compound SM2 is added, for example, stirring is carried out for 30 min-5 h, 30 min-4 h, 30 min-3 h, 30 min-2 h, 1 h-2 h, or 1 h-1.5 h, or stirring is stopped when HPLC monitoring shows that the content of compound SM2 is less than 2%. In some preferred embodiments, stirring is carried out for 1h-1.5h after the addition of compound SM2.

In some embodiments, the solvent of the mixed solution is an organic solvent, and the organic solvent is preferably one or more of ethyl acetate, tetrahydrofuran, dichloromethane, and chloroform.

In some embodiments, the solvent of the mixed solution is dichloromethane.

In some embodiments, the intermediate solution and/or compound IM2 is obtained under the protection of an inert gas (e.g., nitrogen).

In some embodiments, the mixed solution of triphosgene and 4-dimethylaminopyridine is obtained by the following method:
4-dimethylaminopyridine is dissolved in an organic solvent, and a triphosgene solution dissolved in an organic solvent is added thereto, stirring is carried out to obtain the mixed solution.

In some embodiments, the triphosgene solution dissolved in an organic solvent is added within the range of ±25 °C, such as within the range of ±20 °C, within the range of ±15 °C, within the range of ±10 °C, or within the range of ±5 °C.

In some embodiments, 4-dimethylaminopyridine is dissolved in 2-50 times, for example 5-50 times, 10-45 times, 10-40 times, 10-35 times, 10-30 times, 10-25 times, 10-20 times, or 10-15 times by weight of the organic solvent.

In some embodiments, the weight ratio of triphosgene to the organic solvent in the triphosgene solution is 1:(10-50), for example 1:(10-40), 1:(10-30), 1:(10-20), 1:(20-50), 1:(20-40), 1:(20-30), 1:(30-50), 1:(30-40), or 1:(40-50).

In some embodiments, the mixture is stirred for 5 min-2 h, 5 min-1.5 h, 5 min-1 h, 5 min-30 min, 5 min-20 min, 5 min-10 min, 10 min-30 min, or 10 min-20 min to obtain the mixed solution of triphosgene and 4-dimethylaminopyridine.

In some embodiments, the organic solvent is one or more of ethyl acetate, tetrahydrofuran, dichloromethane, and chloroform.

In some embodiments, the mixed solution of triphosgene and 4-dimethylaminopyridine is obtained by the following method:
dissolving 4-dimethylaminopyridine in dichloromethane, adding a dichloromethane solution of triphosgene within the range of ±25 °C, and stirring the mixture for 5min-2h to obtain the mixed solution.

In some embodiments, the dichloromethane solution of triphosgene is added within the range of ±20 °C, within the range of ±15 °C, within the range of ±10 °C, or within the range of ±5 °C. In some preferred embodiments, the dichloromethane solution of triphosgene is added within the range of <10 °C. In some embodiments, 4-dimethylaminopyridine is dissolved in 10-50 times, for example, 10-45 times, 10-40 times, 10-35 times, 10-30 times, 10-25 times, 10-20 times, or 10-15 times by weight of dichloromethane.

In some embodiments, the weight ratio of triphosgene to dichloromethane in the dichloromethane solution of triphosgene is 1:(10-50), for example 1:(10-40), 1:(10-30), 1:(10-20), 1:(20-50), 1:(20-40), 1:(20-30), 1:(30-50), 1:(30-40), or 1:(40-50).

In some embodiments, stirring is carried out for 5min-1.5h, 5min-1h, 5min-30min, 5min-20min, 5min-10min, 10min-30min, or 10min-20min to obtain the mixed solution.

In some embodiments, compound IM2 is purified by normal phase chromatography.

The above preparation method can significantly improve the conversion rate of the expensive material SM1 by adjusting the addition order of the reaction raw materials and optimizing the reaction conditions. No drastic or significant temperature increase occurs during the production process, which reduces production risks and improves process stability and scalability.

In another aspect, the present application provides a method suitable for industrial preparation of compound IM3, which comprises the step of preparing compound IM2 as described above; wherein compound IM3 has the structure shown below:

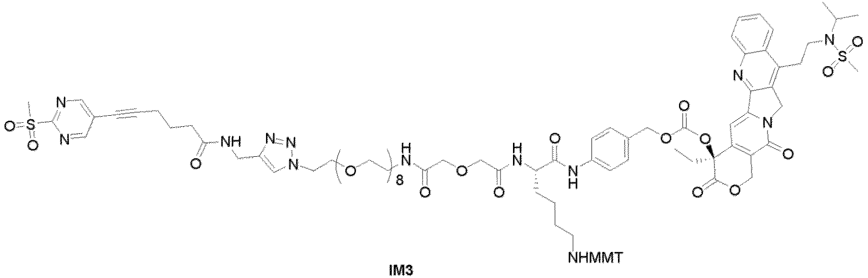

In some embodiments, the method further comprises the following steps:
adding compounds IM2 and SM3 to a mixed solvent of an organic solvent and H₂O, and then adding CuBr, stirring the mixture to obtain compound IM3; wherein compound SM3 has the following structure: 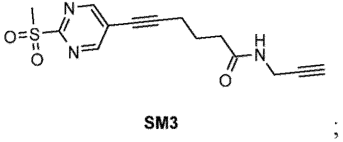
the organic solvent is selected from one or more of DMSO, DMF, DMA and NMP.

In some embodiments, the volume ratio of the organic solvent to H₂O is (2-8): 1, preferably (3-7): 1.

In some embodiments, the method further comprises the following steps:
adding compounds IM2 and SM3 to a mixed solvent of DMSO and H₂O, and then adding CuBr, stirring the mixture to obtain compound IM3; wherein compound SM3 has the following structure:

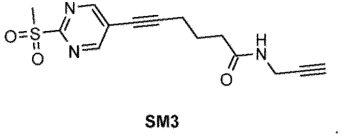

Taking into account the solubility of DMSO and CuBr, the doses of DMSO and H₂O need to be adjusted within a certain ratio range. In some embodiments, the volume ratio of DMSO to H₂O is (4-6):1, preferably (4.5-6): 1, such as 4.5:1, 5:1, 5.5:1, or 6:1, preferably 4.5:1. In some embodiments, the weight ratio of compound IM2 to the mixed solvent is 1:(1-15), preferably 1:(1-12).

In some embodiments, the weight ratio of compound IM2 to the mixed solvent is 1:(2-10), for example 1:(2-9), 1:(2-8), 1:(2-7), 1:(2-6), 1:(2-5), 1:(2-4), 1:(2-3), 1:(3-10), 1:(3-9), 1:(3-8), 1:(3-7), 1:(3-6), 1:(3-5), 1:(3-4), 1:(4-10), 1:(4-9), 1:(4-8), 1:(4-7), 1:(4-6), 1:(4-5), 1:(5-10), 1:(5-9), 1:(5-8), 1:(5-7), 1:(5-6), 1:(6-10), 1:(6-9), 1:(6-8), 1:(6-7), 1:(7-10), 1:(7-9), 1:(7-8), 1:(8-10), 1:(8-9), 1:(9-10).

In some embodiments, the molar ratio between compound IM2 and SM3 is 1:(0.2-4), preferably 1:(0.5-2).

In some embodiments, the molar ratio between compounds IM2 and SM3 is 1:(1-2), such as 1:1, 1:1.2, 1:1.4, 1:1.6, 1:1.8, or 1:2.

In some embodiments, compound IM2 is added first, stirred until dissolved, and then compound SM3 is added.

In some embodiments, compounds IM2 and SM3 are added at 15-40 °C.

In some embodiments, compounds IM2 and SM3 are added at 20-30 °C.

In some embodiments, the stirring operation is performed at 15-40 °C; preferably, the stirring is performed for 15 min-4 h, such as 0.5-3 h, or the stirring is stopped when HPLC monitoring shows that the content of compound IM2 is less than 2.0%.

In some embodiments, the stirring operation is performed at 20-30 °C; preferably, the stirring is performed for 30min-2h, such as 1-1.5h, or the stirring is stopped when HPLC monitoring shows that the content of compound IM2 is less than 2.0%.

In some embodiments, the compound IM3 is prepared under the protection of an inert gas (such as nitrogen).

In some embodiments, after the stirring, it further includes the step of post-treating the obtained reaction mixture:
the reaction mixture is diluted with an organic solvent, washed with an aqueous EDTA-2Na solution at pH 4-8, and the organic phase is collected; the organic solvent is selected from one or more of ethyl acetate, dichloromethane, and chloroform.

In some embodiments, the amount of the organic solvent used to dilute the reaction mixture is 10-50 times, for example, 10 times, 20 times, 30 times, 40 times, or 50 times by weight of the feed amount of compound IM2.

In some embodiments, after the reaction mixture is diluted with an organic solvent, it is washed with an aqueous EDTA-2Na solution at pH 5.5-8.0, for example, with an aqueous EDTA-2Na solution at pH 5.5-6.0, 6.5-7.0, or 7.5-8.0; preferably, the aqueous EDTA-2Na solution is an aqueous EDTA-2Na solution with a weight percentage of 3-8%; further preferably, the aqueous EDTA-2Na solution is an aqueous EDTA-2Na solution with a weight percentage of 4%.

In some embodiments, the organic phase obtained after washing with an aqueous EDTA-2Na solution is washed 1-2 times (e.g., 1 time) with an aqueous EDTA-2Na solution at pH 5.5-8.0 (preferably a 4% aqueous EDTA-2Na solution) and 1-2 times (e.g., 2 times) with an aqueous NaCl solution (preferably a 10% aqueous NaCl solution), respectively.

In some embodiments, after the obtained organic phase is washed with an aqueous NaCl solution, it further includes the steps of drying, further washing with an organic solvent for 1-2 times, and concentrating; the organic solvent is preferably one or more of ethyl acetate, dichloromethane, and chloroform, further preferably dichloromethane; preferably, in each washing, the amount of the organic solvent used is equivalent to 10-20 times, for example, 10 times, 12 times, 14 times, 16 times, 18 times, or 20 times by weight of the feed amount of compound IM2.

In some embodiments, after the stirring, it further includes the step of post-treating the obtained reaction mixture:
the reaction mixture is diluted with dichloromethane, washed with an aqueous EDTA-2Na solution at pH 4-8, and the organic phase is collected.

In some embodiments, the amount of dichloromethane used to dilute the reaction mixture is 10-50 times, for example, 10 times, 20 times, 30 times, 40 times, or 50 times by weight of the feed amount of compound IM2.

In some embodiments, the amount of the aqueous EDTA-2Na solution is 10-50 times, for example, 10 times, 20 times, 30 times, 40 times or 50 times by weight of the feed amount of compound IM2.

Experiments show that pre-adjusting the pH of EDTA-2Na with an aqueous NaHCO₃ solution will improve the stability of the product IM3 in solution. In some embodiments, after diluted with dichloromethane, the reaction mixture is washed with a 4% aqueous EDTA-2Na solution at pH 5.5-8.0, for example, with a 4% aqueous EDTA-2Na solution at pH 5.5-6.0, 6.5-7.0, or 7.5-8.0.

In some embodiments, the obtained organic phase is washed 1-2 times with a 4% aqueous EDTA-2Na solution at pH 5.5-8.0, and 1-2 times with an aqueous NaCl solution (preferably a 10% aqueous NaCl solution), respectively.

In some embodiments, the obtained organic phase is washed 1 time with a 4% aqueous EDTA-2Na solution at pH 5.5-8.0, and 2 times with a 10% aqueous NaCl solution, respectively.

In some embodiments, when washing, the amount of the 4% aqueous EDTA-2Na solution at pH5.5-8.0 is 10-50 times, for example, 10 times, 20 times, 30 times, 40 times, or 50 times, preferably 10 times by weight of the feed amount of compound IM2.

In some embodiments, in each washing, the amount of the aqueous NaCl solution is 10-50 times, for example, 10 times, 20 times, 30 times, 40 times, or 50 times by weight of the feed amount of compound IM2. In some preferred embodiments, in the first washing, the amount of the 10% aqueous NaCl solution used is equivalent to 10-20 times by weight of the feed amount of compound IM2; in the second washing, the amount of the 10% aqueous NaCl solution used is equivalent to 20-30 times by weight of the feed amount of compound IM2.

In some embodiments, after the obtained organic phase is subjected to the washing treatment described above, it further comprises the steps of drying, further washing with dichloromethane for 1-2 times, and concentrating. In some embodiments, in each washing, the amount of dichloromethane is 10-20 times, for example, 10 times, 12 times, 14 times, 16 times, 18 times or 20 times by weight of the feed amount of compound IM2.

In some embodiments, the above-mentioned washing operations are all performed at 5-25 °C, such as 5-20 °C, preferably 5-15 °C. In another aspect, the present application provides a method for preparing compound I, which comprises the aforementioned steps of preparing compound IM2; and/or
the aforementioned steps for preparing compound IM3;
wherein, compound I has the following structure:

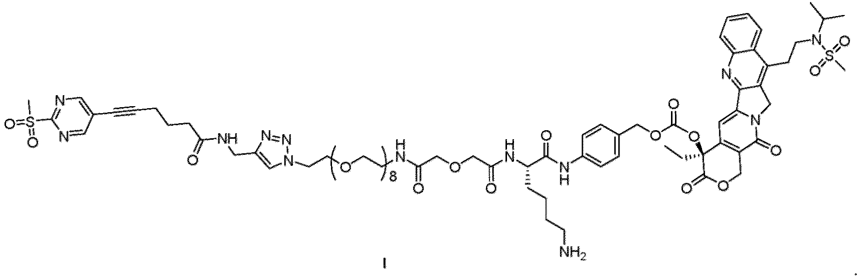

In some embodiments, the method further comprises the following steps:
deprotecting compound IM3 with an acid to obtain compound I; the acid is selected from trifluoroacetic acid, acetic acid, hydrochloric acid or sulfuric acid; preferably trifluoroacetic acid.

In some embodiments, compound IM3 is dissolved in 30-100 times, for example, 40-60 times, 50-70 times, 50-80 times, or 50-90 times of an organic solvent by weight; the organic solvent is preferably one or more of ethyl acetate, dichloromethane, and chloroform, further preferably dichloromethane.

In some embodiments, the molar ratio of compound IM3 to the acid is 1:(5-20), such as 1:(5-10), 1:(5-15), 1:(10-15), or 1:(10-20).

In some embodiments, the method further comprises the following steps:
deprotecting compound IM3 with TFA to give compound I.

In some embodiments, compound IM3 is dissolved in 50-100 times, for example, 50-60 times, 50-70 times, 50-80 times, or 50-90 times by weight of dichloromethane.

In some embodiments, the molar ratio of compound IM3 to TFA is 1:(5-20), such as 1:(5-10), 1:(5-15), 1:(10-15), or 1:(10-20).

In some embodiments, the deprotection is carried out at 0-25 °C, such as 5-10 °C.

In some embodiments, the deprotection is performed under the protection of an inert gas (e.g., nitrogen).

In some embodiments, the deprotection is carried out for 30 min-24 h, such as 4-5 h, or the reaction is terminated when HPLC monitoring shows that the content of compound IM3 is less than 1.0%.

In the preparation method described in any of the above aspects, the production scale of IM1, IM2 and IM3 is above g level, for example, above 10g level, for example, above 100g level, for example, above 1Kg level, for example, above 10Kg level, for example, above 100Kg level, for example, above It level. In some preferred embodiments, the weight of each of SM1, SM2, IM1, IM2, and IM3 is greater than 10g, for example ≥20g, ≥25g, ≥30g, ≥35g, ≥40g, ≥50g, ≥60g, ≥70g, ≥80g, ≥90g, ≥100g, ≥120g, ≥150g, ≥200g, ≥250g, and ≥300g.

In another aspect, the present application provides a method for preparing compound SM1, which comprises a step of reacting belotecan hydrochloride with methanesulfonyl chloride.

In some embodiments, the step is performed in the presence of an alkaline reagent and an organic solvent.

In some embodiments, the alkaline reagent is triethylamine.

In some embodiments, the organic solvent is dichloromethane. In some embodiments, the step is performed at room temperature.

In some embodiments, the step is performed for 0.5-5 h, such as 1-4 h, 2-4 h, 2-3 h, 2 h.

In another aspect, the present application provides a method for preparing compound SM3, which includes a step of reacting prop-2-yn-1-amine and 6-(2-(methylsulfonyl)pyrimidin-5-yl)-5-hexynoic acid.

In some embodiments, the reaction of the step is carried out in the presence of N,N-diisopropylethylamine and O-(7-azabenzotriazol-1-yl)-N,N,N',N'-tetramethyluronium hexafluorophosphate.

In some embodiments, the reaction solvent of the step is dichloromethane.

In some embodiments, the reaction time of the step is 0.5-5 h, such as 1-4 h, 2-4 h, 2-3 h, 2 h.

In another aspect, the present application provides a method for preparing 6-(2-(methylsulfonyl)pyrimidin-5-yl)-5-hexynoic acid, the method comprising a step (c) of reacting 6-(2-(methylthio)pyrimidin-5-yl)-5-hexynoic acid with meta-chloroperbenzoic acid.

In some embodiments, the step (c) is performed in dichloromethane.

In some embodiments, the step (c) is performed at room temperature.

In some embodiments, the method further comprises a step (b) of preparing 6-(2-(methylthio)pyrimidin-5-yl)-5-hexynoic acid from methyl 6-(2-(methylthio)pyrimidin-5-yl)-5-hexynoate.

In some embodiments, the step (b) is performed in the presence of lithium hydroxide monohydrate.

In some embodiments, the step (b) is performed in a mixed solution of tetrahydrofuran and water, for example, a mixed solution of tetrahydrofuran and water in a volume ratio of 1:1.

In some embodiments, the step (b) is performed at room temperature.

In some embodiments, the method further comprises a step (a) of preparing methyl 6-(2-(methylthio)pyrimidin-5-yl)-5-hexynoate from methyl 5-hexynoate and 5-bromo-2-methylthiopyrimidine.

In some embodiments, the step (a) is performed in the presence of triethylamine, cuprous iodide and ditriphenylphosphine palladium dichloride.

In some embodiments, the step (a) is performed in N,N-dimethylformamide.

In some embodiments, the step (a) is performed under the protection of nitrogen.

In some embodiments, the step (a) is performed at 60-120°C, such as 70-110°C, 80-105°C, 85-100°C, 90-95°C, 95°C.

In some embodiments, the step (a) is performed for 1-10 h, such as 3-9 h, 5-8 h, 6-7 h, 6 h.

In another aspect, the present application provides a compound for preparing an antibody-drug conjugate, which is selected from:
(i) a compound of formula I;
(ii) SM1, SM2 or SM3;
(iii) IM1, IM2 or IM3;
(iv) 6-(2-(methylsulfonyl)pyrimidin-5-yl)-5-hexynoic acid;
(v) 6-(2-(methylthio)pyrimidin-5-yl)-5-hexynoic acid;
(vi) methyl 6-(2-(methylthio)pyrimidin-5-yl)-5-hexynoate; or
(vii) 5-bromo-2-methylthiopyrimidine.

In another aspect, the present application provides the use of a compound in the preparation of an antibody-drug conjugate, wherein the compound is selected from:
(i) a compound of formula I;
(ii) SM1, SM2 or SM3;
(iii) IM1, IM2 or IM3;
(iv) 6-(2-(methylsulfonyl)pyrimidin-5-yl)-5-hexynoic acid;
(v) 6-(2-(methylthio)pyrimidin-5-yl)-5-hexynoic acid;
(vi) methyl 6-(2-(methylthio)pyrimidin-5-yl)-5-hexynoate; or
(vii) 5-bromo-2-methylthiopyrimidine.

In another aspect, the present application provides a compound for preparing a drug-linker conjugate shown in formula I, which is selected from:
(i) SM1, SM2 or SM3;
(ii) IM1, IM2 or IM3;
(iii) 6-(2-(methylsulfonyl)pyrimidin-5-yl)-5-hexynoic acid;
(iv) 6-(2-(methylthio)pyrimidin-5-yl)-5-hexynoic acid;
(v) methyl 6-(2-(methylthio)pyrimidin-5-yl)-5-hexynoate; or
(vi) 5-bromo-2-methylthiopyrimidine.

In another aspect, the present application provides the use of a compound in the preparation of a drug-linker conjugate shown in formula I, wherein the compound is selected from:
(i) SM1, SM2 or SM3;
(ii) IM1, IM2 or IM3;
(iii) 6-(2-(methylsulfonyl)pyrimidin-5-yl)-5-hexynoic acid;
(iv) 6-(2-(methylthio)pyrimidin-5-yl)-5-hexynoic acid;
(v) methyl 6-(2-(methylthio)pyrimidin-5-yl)-5-hexynoate; or
(vi) 5-bromo-2-methylthiopyrimidine.

In another aspect, the present application provides a compound for preparing a linker compound in an antibody-drug conjugate, which is selected from:
(i) SM2 or SM3;
(ii) 6-(2-(methylsulfonyl)pyrimidin-5-yl)-5-hexynoic acid;
(iii) 6-(2-(methylthio)pyrimidin-5-yl)-5-hexynoic acid;
(iv) methyl 6-(2-(methylthio)pyrimidin-5-yl)-5-hexynoate; or
(v) 5-bromo-2-methylthiopyrimidine.

In another aspect, the present application provides the use of a compound in the preparation of a linker compound in an antibody-drug conjugate, wherein the compound is selected from:
(i) SM2 or SM3;
(ii) 6-(2-(methylsulfonyl)pyrimidin-5-yl)-5-hexynoic acid;
(iii) 6-(2-(methylthio)pyrimidin-5-yl)-5-hexynoic acid;
(iv) methyl 6-(2-(methylthio)pyrimidin-5-yl)-5-hexynoate; or
(v) 5-bromo-2-methylthiopyrimidine.

In another aspect, the present application provides a compound for preparing SM3, which is selected from:
(i) 6-(2-(methylsulfonyl)pyrimidin-5-yl)-5-hexynoic acid;
(ii) 6-(2-(methylthio)pyrimidin-5-yl)-5-hexynoic acid;
(iii) methyl 6-(2-(methylthio)pyrimidin-5-yl)-5-hexynoate; or
(iv) 5-bromo-2-methylthiopyrimidine.

In another aspect, the present application provides the use of a compound in the preparation of SM3, wherein the compound is selected from:
(i) 6-(2-(methylsulfonyl)pyrimidin-5-yl)-5-hexynoic acid;
(ii) 6-(2-(methylthio)pyrimidin-5-yl)-5-hexynoic acid;
(iii) methyl 6-(2-(methylthio)pyrimidin-5-yl)-5-hexynoate; or
(iv) 5-bromo-2-methylthiopyrimidine.

In another aspect, the present application provides a compound for preparing6-(2-(methylsulfonyl)pyrimidine-5-yl)-5-hexynoic acid, which is selected from:
(i) 6-(2-(methylthio)pyrimidin-5-yl)-5-hexynoic acid;
(ii) methyl 6-(2-(methylthio)pyrimidin-5-yl)-5-hexynoate; or
(iii) 5-bromo-2-methylthiopyrimidine.

In another aspect, the present application provides the use of a compound for preparing6-(2-(methylsulfonyl)pyrimidine-5-yl)-5-hexynoic acid, wherein the compound is selected from:
(i) 6-(2-(methylthio)pyrimidin-5-yl)-5-hexynoic acid;
(ii) methyl 6-(2-(methylthio)pyrimidin-5-yl)-5-hexynoate; or
(iii) 5-bromo-2-methylthiopyrimidine.

### Beneficial Effects of the Invention

The present application provides a method for preparing a drug-linker conjugate shown in formula I and its intermediates IM2 and IM3. The stability, practicality and scalability of the process are improved by optimizing the preparation process of IM2 and using a normal chromatography purification method that is easily scalable.

### DETAILED DESCRIPTION

The technical solutions in the embodiments of the present invention will be described clearly and completely below in conjunction with the drawings in the embodiments of the present invention. Obviously, the described embodiments are only part of the embodiments of the present invention, rather than all the embodiments. The following description of at least one exemplary embodiment is merely illustrative in nature and is in no way intended to limit the present invention, its application, or uses. Based on the embodiments of the present invention, all other embodiments obtained by ordinary technicians in this field without making any creative work shall fall within the scope of protection of the present invention.

If no specific conditions are specified in the embodiments, the experiments are carried out according to conventional conditions or conditions recommended by the manufacturer. The reagents and instruments used without indicating the manufacturer are all conventional products that can be purchased commercially.

Abbreviations herein have the following meanings:

| Abbreviation | Meaning | Abbreviation | Meaning |
|---|---|---|---|
| DCM | Dichloromethane | EA | Ethyl acetate |
| DMAP | 4-Dimethylaminopyridine | MeOH | Methanol |
| MMT | 4-Methoxytrityl | EDTA-2Na | Disodium EDTA |
| TFA | Trifluoroacetic acid | | |
| DMSO | Dimethyl sulfoxide | DMF | N,N-dimethylformamide |
| DMA | N,N-dimethylacetamide | NMP | N-methylpyrrolidone |

| | | | |
|---|---|---|---|
| Herein, "±a °C" denotes -a°C to a°C, for example ±25 °C denotes -25°C to 25°C. | | | |

### Example 1: Preparation of IM2 (same as step 2 of Example 14 in WO 2019/114666A1)

Under nitrogen protection at 25°C, SM1 (250 mg, 0.49 mmol) was dissolved in dichloromethane (10 mL), cooled to 0 °C, a solution of 4-dimethylaminopyridine (478 mg, 3.91 mmol) in dichloromethane (3 mL) was added, and then a solution of triphosgene (72 mg, 0.24 mmol) in dichloromethane (4 mL) was slowly added dropwise. After the addition was completed, the reaction was stirred at 0°C for 20 min. The conversion of the raw material was monitored by HPLC-MS (the reaction mixture was quenched with methanol and sampled), and the result was 90%. The reaction mixture was purged with nitrogen for 20 min and used directly in the next step without purification. A solution of SM2 (518 mg, 0.49 mmol) in dichloromethane (7 mL) was added to the reaction mixture. After the addition was completed, the mixture was stirred at 0°C for 1 h. The conversion of the raw material was monitored by HPLC-MS/MS chromatography, showing complete conversion. The system was purified by preparative HPLC to obtain 500 mg of IM2, a light yellow solid product with a yield of 64%.

The disadvantage of the above example is that when the dichloromethane solution of triphosgene is added dropwise, the temperature rises significantly, which makes the scaled-up production have a great risk. As a result of the temperature rise, the stability of the intermediate in this system is particularly poor.

### Experimental Example 2: Preparation of IM2

In a 2L glass reaction flask, N₂ replacement was carried out, 927.50 g of DCM was added, and then 70.00 g of DMAP was added, and the mixture was stirred to dissolve. The temperature was lowered to 0±5°C, and triphosgene DCM solution (20.30 g triphosgene/621.60 g DCM) was slowly added dropwise to the reaction flask. After the addition was completed, the system was further stirred o react at -5°C to 5°C, then 70.00 g of SM1 was added, and then a DCM solution of SM2 (144.90 g SM2/927.50 g DCM) was added. The stirring was continued for 1-1.5 h. The content of SM2 in the reaction mixture was detected by HPLC and was less than 2.0%. Workup: 2786.00g of dichloromethane was added to the reaction flask for dilution, then the system was washed twice with 4.41Kg*2 5.0% aqueous NaCl solution, stirred for 15-20min each time. The layers were separated, 700.00g of anhydrous sodium sulfate was added to the DCM phase for drying with stirring, the mixture was filtered, and concentrated under reduced pressure until no solvent was obviously distilled out to obtain a crude product. The crude product was purified by column chromatography to obtain IM2 with a yield of 55.0-75.0% and a purity of >95.0%.

DMAP was dissolved in DCM, which was followed by the addition of a dichloromethane solution of triphosgene, and then followed by the addition of SM1. This addition sequence improved the stability and scalability of the process.

Through the process of the above example, the reaction can be safely scaled up to 200g (SM1 feed amount).

### Example 3: Screening of the amount of triphosgene used in the preparation of IM2

5 reaction flasks were taken, and subjected to N₂ replacement, then 99.82mg of DMAP and 1mL of dichloromethane were added, and after dissolving, a solution of triphosgene (the molar ratio of triphosgene to SM1 in groups A, B, C, D and E is 0.3, 0.4, 0.5, 0.7 and 1.0, respectively) in 0.67ml of dichloromethane was added, the internal temperature was controlled not higher than 10°C, then 100mg of SM1 was added, and the reaction mixture after reacting for 15min was detected by high performance liquid chromatography. The contents of SM1 and IM1 in the reaction mixture are as follows:

| Group No. | Molar ratio of triphosgene to SM1 | SM1 | IM1 |
|---|---|---|---|
| A | 0.3 | 40.3% | 55.03% |
| B | 0.4 | 6.78% | 88.23% |
| C | 0.5 | 0.71% | 96.25% |
| D | 0.7 | 0.74% | 97.49% |
| E | 1.0 | 97.28% | 1.70% |

When the triphosgene content is low (molar ratio of 0.3) or high (molar ratio of 1.0), the conversion of the starting material from SM1 to the active intermediate IM1 is inhibited, and the conversion rate is poor.

### Example 4: Screening of the amount of IM2 used to prepare DMAP

5 reaction flasks were taken, and subjected to N₂ replacement, then DMAP (the molar ratios of DMAP to SM1 in group A, group B, group C and group D are 3.0, 3.7, 4.18, 4.5 and 5.0, respectively) and 1mL of dichloromethane were added, and after dissolving, a solution of 29 mg of triphosgene in 0.67ml of dichloromethane was added, the internal temperature was controlled not higher than 10°C, then 100mg of SM1 was added, and the reaction mixture after reacting for 15min was detected by high performance liquid chromatography. The contents of SM1 and IM1 in the reaction mixture are as follows:

| Group No. | Molar ratio of DMAP to SM1 | SM1 | IM1 |
|---|---|---|---|
| A | 3.0 | 96.13% | 0.90% |
| B | 3.7 | 0.24% | 96.84% |
| C | 4.18 | 0.18% | 96.99% |
| D | 4.5 | 0.47% | 94.79% |
| E | 5.0 | 7.96% | 86.71% |

When the DMAP content is low (3.0eq), a large amount of SM1 cannot be converted to generate the active intermediate IM1 for preparing IM2. When the DMAP concentration increases significantly (5.0eq), the conversion rate of SM1 decreases significantly, and the corresponding IM1 also decreases.

### Experimental Example 5: Preparation of IM3

A 1L reaction flask was subjected to N₂ replacement, 687.45g of DMSO and 124.95g of H₂O were added, stirring was started, the temperature was controlled to 20-30°C, 150.00g of IM2 was slowly added, the system was stirred until dissolved, then 31.50g of SM3 was added, the system was stirred until dissolved, 20.25g of CuBr was added, the reaction temperature was controlled to 20-30°C, and the reaction was continued for 1h-1.5h. Workup: the reaction mixture was diluted with 2981.25 g of DCM, 3120.00 g of a 4% aqueous EDTA-2Na solution (pre-adjusted to pH = 6.5-7.0 with NaHCO₃) was added, the system was washed and stirred, the washing temperature was controlled at 5-15°C, and the system was allowed to stand for 5-10 min. The organic phase was washed and stirred with 1560.00g of 4% EDTA-2Na (pre-adjusted to pH = 6.5-7.0 with NaHCO₃) and 1650.00 g of 10% NaCl respectively, and the washing temperature was controlled at 5-15°C. The organic phase was then washed with 3300.00 g of 10% NaCl with stirring, with the washing temperature controlled at 5-15°C. It was dried over 1500 g of anhydrous Na₂SO₄, stirred for 30 min, filtered, and washed twice with 994.50 g*2 DCM. The system was concentrated under reduced pressure at 30-35°C to remove DCM to obtain IM3.

Through the process of the above example, the reaction can be safely scaled up to 370g (IM2 feed amount).

### Example 6: Optimization of the ratio between solvents DMSO and H₂O in the preparation of IM3

After N₂ replacement, 1 ml of a mixed solvent of DMSO and H₂O (the volume ratio of DMSO to H₂O in group A, group B, group C and group D is 4:1, 4.5:1, 5:1 and 6:1, respectively) was added to the reaction flask, then 200 mg of IM2 was added, after the mixture was stirred and dissolved (it was not completely dissolved in group A), 42 mg of SM3 was added, the mixture was stirred and dissolved (it was not completely dissolved in group A), and then 26 mg of cuprous bromide was added, N2 replacement was performed, and the reaction was carried out at 20-30°C for 1.5 h. The reaction mixture was detected by HPLC, and the contents of SM3 and IM2 in the reaction mixture are as follows:

| Group No. | DMSO/H₂O (volume ratio) | SM3 | IM3 |
|---|---|---|---|
| A | 4:1 | 13.13% | 76.27% |
| B | 4.5:1 | 0.05% | 88.60% |
| C | 5:1 | 0.44% | 85.44% |
| D | 6:1 | 4.43% | 83.79% |

The HPLC contents of IM3 in groups A, B, C, and D are 76.27%, 88.60%, 85.44%, and 83.79%, respectively. When the volume ratio of DMSO to H₂O is lower than 4.5:1, the conversion rate of SM3 decreases significantly.

### Example 7: pH range of aqueous EDTA-2Na solution in IM3 workup

The IM3 reaction mixture diluted with DCM in Example 5 was washed with 4% aqueous EDTA-2Na solution of the following pH values (pH was adjusted with NaHCO₃), and the reaction mixture after washing was detected by HPLC. The content of IM3 in the reaction mixture is as follows:

| Group No. | pH of aqueous EDTA-2Na solution | IM3 |
|---|---|---|
| A | 4.2-4.4 | 80.73% |
| B | 5.5-6.0 | 87.06% |
| C | 6.5-7.0 | 87.06% |
| D | 7.5-8.0 | 86.59% |

The above example shows that after washing at pH 4.2-4.4 (without adding NaHCO₃), IM3 is degraded by about 6%. When the pH of the aqueous EDTA-2Na solution is adjusted to 5.5-8.0 with NaHCO₃, IM3 has good stability.

### Example 8 Preparation of compound I

Under N₂ atmosphere, 8.18 kg of dichloromethane was added into a reaction flask. IM3 was quickly added in the dark. Then, a dichloromethane solution of trifluoroacetic acid (83.54 g of trifluoroacetic acid dissolved in 258.34 g of dichloromethane) was added dropwise into the reaction flask, and the reaction system was controlled at 5 - 10°C during the addition process. After stirring for 4-5 h, the reaction endpoint was monitored by HPLC. When the IM3 content was less than or equal to 1.0%, the reaction endpoint was reached. After the reaction reached the end point, the reaction mixture was concentrated under vacuum at 30-40°C to remove the solvent to obtain compound I.

### Example 9: Preparation of (S)-N-(2-(4-ethyl-4-hydroxy-3,14-dione-3,4,12,14-tetrahydro-1H-pyrano[3',4',6,7]indolizino[1,2-b]quinolin-11-yl)ethyl)-N-isopropylmethanesulfonamide (SM1)

Methanesulfonyl chloride (462 mg, 12.77 mmol, purity about 70%) was added dropwise to a solution of belotecan hydrochloride (3 g, 6.38 mmol) and triethylamine (2.58 g, 25.54 mmol) in dichloromethane (40 mL), and the mixture was allowed to react at room temperature for 2 h. The reaction mixture was suction-filtered, the filter cake was washed three times with dichloromethane (3 mL) to obtain the title compound, 2.2 g.

The structural characterization data are as follows:
¹H NMR (400 MHz, DMSO-d₆) δ8.32(d,J = 8.4Hz,1H), 8.20(dd,J = 8.4,1.2Hz,1H), 7.93-7.84(m,1H), 7.79(t,J = 7.6Hz, 1H), 7.35(s,1H), 6.56(s,1H), 5.44(d,J = 9.2Hz,4H), 3.98(p,J = 6.7Hz,1H), 3.50(t,J = 8.0Hz,2H), 3.42-3.35(m,2H), 3.00(s,3H), 1.93-1.82(m,2H), 1.15(d,J = 6.7Hz,6H), 0.88(t,J = 7.3Hz,3H). ESI-MS(m/z):512.2[M+H]⁺. [α]_{D}²⁰ is +28.19° (c = 0.101g/100mL, CH₃CN).

### Example 10: Preparation of 6-(2-(methylsulfonyl)pyrimidin-5-yl)-N-(prop-2-yn-1-yl)hex-5-ynamide (SM3)

### Step 1: Synthesis of methyl 6-(2-(methylthio)pyrimidin-5-yl)-5-hexynoate

At room temperature, methyl 5-hexynoate (500 mg, 3.97 mmol) and 5-bromo-2-methylthiopyrimidine were dissolved in N,N-dimethylformamide (3 mL), and triethylamine (3 mL), cuprous iodide (75 mg, 0.4 mmol) and ditriphenylphosphine palladium dichloride (279 mg, 0.4 mmol) were added in sequence. The mixture was heated to 95°C under nitrogen protection and allowed to react with stirring for 6 h, quenched with water, and extracted with ethyl acetate (20 mL × 3). The organic phases were combined, washed with saturated brine (20 mL × 2), dried over anhydrous sodium sulfate, the desiccant was filtered out, the solvent was evaporated under reduced pressure, and the system was purified by preparative liquid chromatography (chromatographic column: Waters SunFire C18 5µm 19x250mm; mobile phase A: acetonitrile; mobile phase B: water containing 0.05% of formic acid; time: 0 min-16 min; mobile phase A: 10%-90%; flow rate: 28mL/min), to obtain the title compound, 300mg. ESI-MS (m/z): 251.3 [M+H]⁺.

### Step 2: Synthesis of 6-(2-(methylthio)pyrimidin-5-yl)-5-hexynoic acid

At room temperature, methyl 6-(2-(methylthio)pyrimidin-5-yl)-5-hexynoate (200 mg, 0.8 mmol) was dissolved in a mixed solution of tetrahydrofuran and water (4 mL/4 mL), and lithium hydroxide monohydrate (235 mg, 5.6 mmol) was added. The reaction was stirred at room temperature for 4 h, diluted with water, extracted with ethyl acetate (20 mL × 2), the aqueous phase was adjusted to pH = 3 with 1N hydrochloric acid, and extracted with ethyl acetate (20 mL × 3). The organic phases were combined, washed with saturated brine (20 mL × 2), dried over anhydrous sodium sulfate, the desiccant was filtered out, and the solvent was evaporated under reduced pressure, thereby obtaining 120 mg of the title compound.

### Step 3: Synthesis of 6-(2-(methylsulfonyl)pyrimidin-5-yl)-5-hexynoic acid

At room temperature, 6-(2-(methylthio)pyrimidin-5-yl)-5-hexynoic acid (20 mg, 0.085 mmol) was dissolved in dichloromethane (4 mL), and m-chloroperbenzoic acid (22 mg, 0.127 mmol) was added. After the addition was completed, the reaction was stirred at room temperature overnight and purified by preparative liquid chromatography (chromatographic column: Waters SunFire C18 5µm 19x250mm; mobile phase A: acetonitrile; mobile phase B: water containing 0.05% of formic acid; time: 0 min-16 min; mobile phase A: 10%-90%; flow rate: 28 mL/min), thereby obtaining 20 mg of the title compound. ESI-MS (m/z): 269.1 [M+H]⁺.

### Step 4: Synthesis of 6-(2-(methylsulfonyl)pyrimidin-5-yl)-N-(prop-2-yn-1-yl)hex-5-ynamide

Prop-2-yn-1-amine (189 mg, 3.4 mmol) and 6-(2-(methylsulfonyl)pyrimidin-5-yl)-5-hexynoic acid (800 mg, 2.83 mmol) were dissolved in dichloromethane (10 mL) at 25 °C, and N,N-diisopropylethylamine (738 mg, 5.67 mmol) and O-(7-azabenzotriazole-1-yl)-N,N,N',N'-tetramethyluronium hexafluorophosphate (1.63 g, 4.25 mmol) were added in sequence, and the reaction was stirred for 2 h. The reaction mixture was concentrated under reduced pressure, and the residue was purified through a flash silica gel column (ethyl acetate/petroleum ether=3/1) to obtain 700 mg of the title compound. ESI-MS (m/z) :306.1[M+H]⁺.

Although the specific embodiment of the present invention has been described in detail, those skilled in the art will understand that according to all the teachings disclosed, various modifications and replacements can be made to those details, and these changes are all within the protection scope of the present invention. The full scope of the invention is given by the appended claims and any equivalents thereof.

## Claims

1. A method suitable for industrial preparation of compound IM2, comprising the following steps:
adding compound SM1 to a mixed solution of triphosgene and 4-dimethylaminopyridine to obtain an intermediate solution;
adding compound SM2 to the intermediate solution and stirring the mixture to obtain compound IM2; wherein,
compounds IM2, SM1 and SM2 have the following structures, respectively:

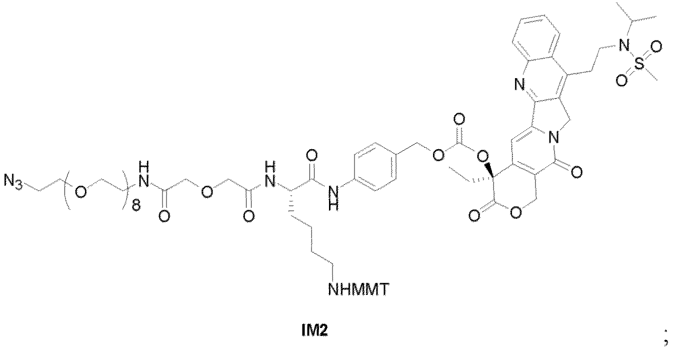

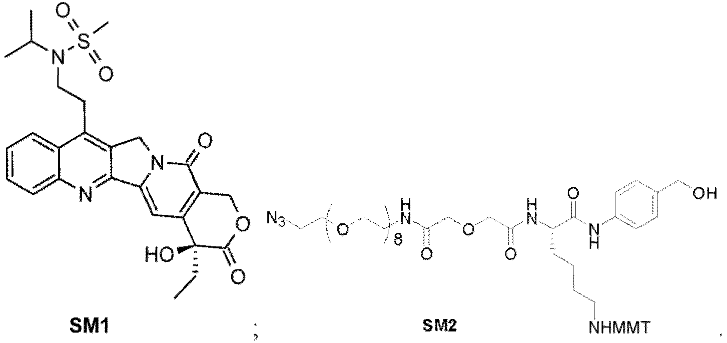

2. The method according to claim 1, **characterized by** one or more of the following:
(I-1) the feed ratio of triphosgene to compound SM1 is <2:1 in terms of molar ratio;
(I-2) the feed ratio of 4-dimethylaminopyridine to compound SM1 is >1:1 in terms of molar ratio;
(I-3) SM1 is added within the range of ±40 °C, preferably SM1 is added within the range of ±30 °C;
(I-4) the feed ratio of SM1 to SM2 is 1:(0.2-4.0) , preferably 1:(0.5-2.0) in terms of molar ratio;
(I-5) after compound SM2 is added, the mixture is stirred for 15 min-8 h;
(I-6) the solvent of the mixed solution is an organic solvent, and the organic solvent is preferably one or more of ethyl acetate, tetrahydrofuran, dichloromethane, and chloroform.

3. The method according to claim 1, **characterized by** one or more of the following:
(I-1) the feed ratio of triphosgene to compound SM1 is <1:1, preferably (0.3-0.9):1, more preferably (0.4-0.7):1, for example 0.4:1, 0.45:1, 0.5:1, 0.55:1, 0.6:1, 0.65:1, or 0.7:1 in terms of molar ratio;
(I-2) the feeding ratio of 4-dimethylaminopyridine to compound SM1 is >3:1, preferably (3.5-5):1, more preferably (3.7-4.5):1, such as 3.7:1, 3.8:1, 3.9:1, 4.0:1, 4.1:1, 4.2:1, 4.3:1, 4.4:1, or 4.5:1 in terms of molar ratio;
(I-3) SM1 is added within the range of ±25 °C, such as within the range of ±20 °C, within the range of ±15 °C, within the range of ±10 °C, or within the range of ±5 °C;
(I-4) the feed ratio of SM1 to SM2 is 1:(1-2.0), for example 1:1.1, 1:1.2, 1:1.3, 1:1.4, 1:1.5, 1:1.6, 1:1.7, 1:1.8, 1:1.9, or 1:2.0 in terms of molar ratio;
(I-5) stirring is carried out for 30 min-6 h after compound SM2 is added, for example, stirring is carried out for 30 min-5 h, 30 min-4 h, 30 min-3 h, 30 min-2 h, 1 h-2 h, or 1 h-1.5 h, or stirring is stopped when HPLC monitoring shows that the content of compound SM2 is less than 2%;
(I-6) the solvent of the mixed solution is dichloromethane;
(I-7) the intermediate solution and/or compound IM2 is obtained under the protection of an inert gas (e.g., nitrogen).

4. The method according to any one of claims 1 to 3, wherein the mixed solution of triphosgene and 4-dimethylaminopyridine is obtained by the following method:
4-dimethylaminopyridine is dissolved in an organic solvent, and a triphosgene solution dissolved in an organic solvent is added thereto, stirring is carried out to obtain the mixed solution;
preferably, the triphosgene solution dissolved in an organic solvent is added within the range of ±25 °C, such as within the range of ±20 °C, within the range of ±15 °C, within the range of ±10 °C, or within the range of ±5 °C;
preferably, 4-dimethylaminopyridine is dissolved in 2-50 times, for example, 5-50 times, 10-45 times, 10-40 times, 10-35 times, 10-30 times, 10-25 times, 10-20 times, or 10-15 times by weight of the organic solvent;
preferably, the weight ratio of triphosgene to the organic solvent in the triphosgene solution is 1:(10-50), for example 1:(10-40), 1:(10-30), 1:(10-20), 1:(20-50), 1:(20-40), 1:(20-30), 1:(30-50), 1:(30-40), or 1:(40-50);
preferably, stirring is carried out for 5 min-2 h, 5 min-1.5 h, 5 min-1 h, 5 min-30 min, 5 min-20 min, 5 min-10 min, 10 min-30 min, or 10 min-20 min to obtain the mixed solution;
preferably, the organic solvent is one or more of ethyl acetate, tetrahydrofuran, dichloromethane, and chloroform.

5. The method according to any one of claims 1 to 4, wherein the mixed solution of triphosgene and 4-dimethylaminopyridine is obtained by the following method:
dissolving 4-dimethylaminopyridine in dichloromethane, adding a dichloromethane solution of triphosgene within the range of ±25 °C, and stirring the mixture for 5min-2h to obtain the mixed solution;
preferably, the dichloromethane solution of triphosgene is added within the range of ±20 °C, within the range of ±15 °C, within the range of ±10 °C, or within the range of ±5 °C;
preferably, 4-dimethylaminopyridine is dissolved in 10-50 times, for example, 10-45 times, 10-40 times, 10-35 times, 10-30 times, 10-25 times, 10-20 times, or 10-15 times, by weight of dichloromethane;
preferably, the weight ratio of triphosgene to dichloromethane in the dichloromethane solution of triphosgene is 1:(10-50), for example 1:(10-40), 1:(10-30), 1:(10-20), 1:(20-50), 1:(20-40), 1:(20-30), 1:(30-50), 1:(30-40), or 1:(40-50);
preferably, stirring is carried out for 5min-1.5h, 5min-1h, 5min-30min, 5min-20min, 5min-10min, 10min-30min, or 10min-20min to obtain the mixed solution.

6. A method suitable for industrial preparation of compound IM3, comprising a step of preparing compound IM2 by the method according to any one of claims 1 to 5; wherein compound IM3 has a structure shown below:

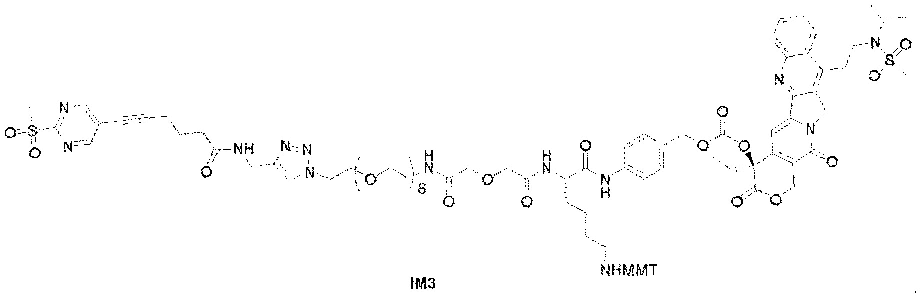

7. The method according to claim 6, further comprising the following steps:
adding compounds IM2 and SM3 to a mixed solvent of an organic solvent and H₂O, and then adding CuBr, stirring the mixture to obtain compound IM3; wherein compound SM3 has the following structure: 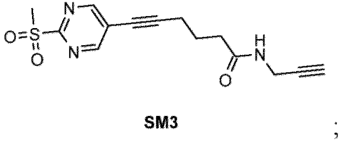
the organic solvent is selected from one or more of DMSO, DMF, DMA and NMP.

8. The method according to claim 6, further comprising the following steps:
adding compounds IM2 and SM3 to a mixed solvent of DMSO and H₂O, and then adding CuBr, stirring the mixture to obtain compound IM3; wherein compound SM3 has the following structure:

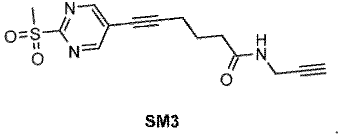

9. The method according to any one of claim 7 or 8, **characterized by** one or more of the following:
(II-1) the volume ratio of the organic solvent to H₂O is (2-8): 1, preferably (3-7): 1;
(II-2) the weight ratio of compound IM2 to the mixed solvent is 1:(1-15), preferably 1:(1-12);
(II-3) the molar ratio of compound IM2 to SM3 is 1:(0.2-4), preferably 1:(0.5-2);
(II-4) the operation of adding compounds IM2 and SM3 is carried out at 15-40 °C;
(II-5) the stirring operation is performed at 15-40 °C; preferably, the stirring is performed for 15 min-4 h, such as 0.5-3 h, or the stirring is stopped when HPLC monitoring shows that the content of compound IM2 is less than 2.0%.

10. The method according to claim 7 or 8, **characterized by** one or more of the following:
(II-1) the volume ratio of DMSO to H₂O is (4-6):1, preferably (4.5-6):1, such as 4.5:1, 5:1, 5.5:1, or 6:1;
(II-2) the weight ratio of compound IM2 to the mixed solvent is 1:(2-10), for example 1:(2-9), 1:(2-8), 1:(2-7), 1:(2-6), 1:(2-5), 1:(2-4), 1:(2-3), 1:(3-10), 1:(3-9), 1:(3-8), 1:(3-7), 1:(3-6), 1:(3-5), 1:(3-4), 1:(4-10), 1:(4-9), 1:(4-8), 1:(4-7), 1:(4-6), 1:(4-5), 1:(5-10), 1:(5-9), 1:(5-8), 1:(5-7), 1:(5-6), 1:(6-10), 1:(6-9), 1:(6-8), 1:(6-7), 1:(7-10), 1:(7-9), 1:(7-8), 1:(8-10), 1:(8-9), 1:(9-10);
(II-3) the molar ratio of compounds IM2 and SM3 is 1:(1-2), for example 1:1, 1:1.2, 1:1.4, 1:1.6, 1:1.8, or 1:2;
(II-4) compound IM2 is added first, stirred until dissolved, and then compound SM3 is added;
(II-5) the operation of adding compounds IM2 and SM3 is carried out at 20-30 °C;
(II-6) the stirring operation is performed at 20-30 °C; preferably, the stirring is performed for 30min-2h, such as 1-1.5h, or the stirring is stopped when HPLC monitoring shows that the content of compound IM2 is less than 2.0%;
(II-7) the compound IM3 is prepared under the protection of an inert gas (e.g., nitrogen).

11. The method according to any one of claims 7 to 10, further comprising, after the stirring, a step of workup of the obtained reaction mixture:
the reaction mixture is diluted with an organic solvent, washed with an aqueous EDTA-2Na solution at pH 4-8, and the organic phase is collected; the organic solvent is selected from one or more of ethyl acetate, dichloromethane, and chloroform.

12. The method according to claims 7 to 10, further comprising, after the stirring, a step of workup of the obtained reaction mixture:
the reaction mixture is diluted with dichloromethane, washed with an aqueous EDTA-2Na solution at pH 4-8, and the organic phase is collected.

13. The method according to claim 11, **characterized by** one or more of the following:
(III-1) the amount of the organic solvent used to dilute the reaction mixture is 10-50 times, for example, 10 times, 20 times, 30 times, 40 times, or 50 times by weight of the feed amount of compound IM2;
(III-2) the amount of the aqueous EDTA-2Na solution is 10-50 times, for example, 10 times, 20 times, 30 times, 40 times, or 50 times by weight of the feed amount of compound IM2;
(III-3) after the reaction mixture is diluted with an organic solvent, it is washed with an aqueous EDTA-2Na solution at pH 5.5-8.0, for example, with an aqueous EDTA-2Na solution at pH 5.5-6.0, 6.5-7.0, or 7.5-8.0; preferably, the aqueous EDTA-2Na solution is an aqueous EDTA-2Na solution with a weight percentage of 3-8%; further preferably, the aqueous EDTA-2Na solution is an aqueous EDTA-2Na solution with a weight percentage of 4%;
(III-4) the pH of the EDTA-2Na is adjusted with an aqueous NaHCO₃ solution;
(III-5) the organic phase obtained after washing with an aqueous EDTA-2Na solution is washed 1-2 times (e.g., 1 time) with an aqueous EDTA-2Na solution at pH 5.5-8.0 (preferably a 4% aqueous EDTA-2Na solution) and 1-2 times (e.g., 2 times) with an aqueous NaCl solution (preferably a 10% aqueous NaCl solution), respectively;
preferably, in each washing, the amount of the aqueous EDTA-2Na solution at pH5.5-8.0 is 10-50 times, for example, 10 times, 20 times, 30 times, 40 times, or 50 times by weight of the feed amount of compound IM2;
preferably, in each washing, the amount of the aqueous NaCl solution is 10-50 times, for example, 10 times, 20 times, 30 times, 40 times, or 50 times by weight of the feed amount of compound IM2;
(III-6) after the obtained organic phase is treated as described in (III-5), it further includes the steps of drying, further washing with an organic solvent for 1-2 times, and concentrating; the organic solvent is preferably one or more of ethyl acetate, dichloromethane, and chloroform, further preferably dichloromethane;
preferably, in each washing, the amount of the organic solvent used is equivalent to 10-20 times, for example, 10 times, 12 times, 14 times, 16 times, 18 times, or 20 times by weight of the feed amount of compound IM2;
(III-7) each washing operation is carried out at 5-25 °C, for example 5-20 °C, preferably 5-15 °C.

14. The method according to claim 12, **characterized by** one or more of the following:
(III-1) the amount of dichloromethane used to dilute the reaction mixture is 10-50 times, for example, 10 times, 20 times, 30 times, 40 times, or 50 times by weight of the feed amount of compound IM2;
(III-2) the amount of the aqueous EDTA-2Na solution is 10-50 times, for example, 10 times, 20 times, 30 times, 40 times, or 50 times by weight of the feed amount of compound IM2;
(III-3) after diluted with dichloromethane, the reaction mixture is washed with a 4% aqueous EDTA-2Na solution at pH 5.5-8.0, for example, with a 4% aqueous EDTA-2Na solution at pH 5.5-6.0, 6.5-7.0, or 7.5-8.0;
(III-4) the pH of the EDTA-2Na is adjusted with an aqueous NaHCO₃ solution;
(III-5) the obtained organic phase is washed with a 4% aqueous EDTA-2Na solution at pH 5.5-8.0 for 1-2 times (e.g., 1 time), and an aqueous NaCl solution (preferably a 10% aqueous NaCl solution) for 1-2 times (e.g., 2 times), respectively;
preferably, in each washing, the amount of the 4% aqueous EDTA-2Na solution at pH5.5-8.0 is 10-50 times, for example, 10 times, 20 times, 30 times, 40 times, or 50 times by weight of the feed amount of compound IM2;
preferably, in each washing, the amount of the aqueous NaCl solution is 10-50 times, for example, 10 times, 20 times, 30 times, 40 times, or 50 times by weight of the feed amount of compound IM2;
(III-6) after the obtained organic phase is treated as described in (III-5), it further comprises the steps of drying, further washing with dichloromethane for 1-2 times, and concentrating;
preferably, in each washing, the amount of dichloromethane used is equivalent to 10-20 times, for example, 10 times, 12 times, 14 times, 16 times, 18 times, or 20 times by weight of the feed amount of compound IM2;
(III-7) each washing operation is carried out at 5-25 °C, for example 5-20 °C, preferably 5-15 °C.

15. A method suitable for industrial preparation of compound I, comprising a step of preparing compound IM2 by the method according to any one of claims 1-5; and/or
the step of preparing compound IM3 according to any one of claims 6-14;
wherein, compound I has the following structure:

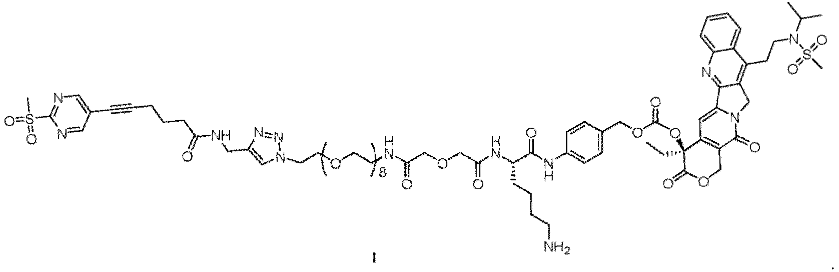

16. The method according to claim 15, further comprising the following step:
deprotecting compound IM3 with an acid to obtain compound I; the acid is selected from trifluoroacetic acid, acetic acid, hydrochloric acid or sulfuric acid; preferably trifluoroacetic acid.

17. The method according to claim 15, further comprising the following step:
deprotecting compound IM3 with TFA to give compound I.

18. The method according to claim 16, **characterized by** one or more of the following:
(IV-1) compound IM3 is dissolved in 30-100 times by weight of an organic solvent, for example 40-60 times, 50-70 times, 50-80 times, or 50-90 times; the organic solvent is preferably one or more of ethyl acetate, dichloromethane, and chloroform, further preferably dichloromethane;
(IV-2) the molar ratio of compound IM3 to the acid is 1:(5-20), for example 1:(5-10), 1:(5-15), 1:(10-15), or 1:(10-20);
(IV-3) the deprotection is carried out at 0-25 °C, for example 5-10 °C;
(IV-4) the deprotection is performed under the protection of an inert gas (e.g., nitrogen);
(IV-5) the deprotection is carried out for 30 min-24 h, such as 4-5 h, or the reaction is terminated when HPLC monitoring shows that the content of compound IM3 is less than 1.0%.

19. The method according to claim 17, **characterized by** one or more of the following:
(IV-1) compound IM3 is dissolved in 50-100 times by weight of dichloromethane, for example 50-60 times, 50-70 times, 50-80 times, or 50-90 times;
(IV-2) the molar ratio of compound IM3 to TFA is 1:(5-20), for example 1:(5-10), 1:(5-15), 1:(10-15), or 1:(10-20);
(IV-3) the deprotection is carried out at 0-25 °C, for example 5-10 °C;
(IV-4) the deprotection is performed under the protection of an inert gas (e.g., nitrogen);
(IV-5) the deprotection is carried out for 30 min-24 h, such as 4-5 h, or the reaction is terminated when HPLC monitoring shows that the content of compound IM3 is less than 1.0%.
